# EUROPEAN PATENT APPLICATION

(11) **EP 2 893 901 A1**
(43) Date of publication of application: **15.07.2015**
(21) Application number: 13834583.0
(22) Date of filing: 06.09.2013
(51) Int. Cl.: A61C 8/00, A61L 27/00

(54) **DENTAL IMPLANT AND METHOD FOR MANUFACTURING SAME**

(30) Priority: 07.09.2012 JP 2012197936
(71) Applicant: Kabushiki Kaisya Advance, Tokyo 103-8354 (JP)
(72) Inventor: URAKABE, Nobuchika, Tokyo 103-8354 (JP)
(74) Representative: Santarelli
(86) International application number: PCT/JP2013/074122
(87) International publication number: WO 2014/038669

(57) **Abstract**

A dental implant including a columnar core member and a covering layer which is covered over at least part of the outer circumferential surface of the core member and which is comprised of recrystallized apatite-based ceramic, wherein the covering layer is comprised of a hydrothermal treated layer of a plasma spray-deposited covering which is formed by spraying a plasma of a composite containing at least two types of calcium phosphate-based compounds which differ in melting point at the outer circumferential surface of the core member based on the melting point of the low melting point calcium phosphate-based compound among the calcium phosphate-based compounds. This dental implant is provided with a strength enabling it to sufficiently withstand a chewing force over a long period of time and with a fast bondability and affinity with the gum and furthermore is excellent in extraction strength as well.

## Description

### Technical Field

The present invention relates to a dental implant and a method of production of the same. More particularly, the present invention relates to a dental implant which has a covering layer which is comprised of a recrystallized apatite-based ceramic on the outer circumferential surface of a core member (base member) and a method of production of the same. The dental implant of the present invention is provided with a strength which is able to sufficiently withstand repeatedly applied chewing force over a long time period and with fast bondability and affinity with the gum and furthermore is excellent in extraction strength as well, so the placed implant can be securely fastened at an early time, can be securely used over a long period of time without fracture etc., and can also contribute to reduction of costs.

### Background Art

As is well known, a dental implant, also frequently called an "artificial dental root", is designed to replace or repair a natural tooth which has lost its function as a tooth. Its main body is normally comprised of an alveolar bone implant part (root part) which is provided with a screw part and a dental prostheses abutment (support part).

A dental implant, as explained above, uses the alveolar bone implant part and dental prostheses abutment to connect the inside and outside of the body, so it is necessary to prevent bacteria, foreign matter, etc. from outside the body from invading the body and necessary to withstand an average of 300N or so chewing force over a long period of time. Further, the chewing force which is applied to the implant is not applied from a single direction at all times. In addition, hard food constantly strikes the gum, dental prostheses, etc. from various directions, so after placing the implant, fast bonding with the alveolar bone or fast stabilization with the gum positioned at the boundary of the inside and outside of the body is considered required. To solve these problems and to deal with other problems, various types of dental implants have already been proposed.

For example Japanese Patent Publication No. H2-161939A proposes, as an artificial dental root which can withstand biting force due to chewing and other outside pressure and is made to be able to be used over a long time period, a one-piece type of artificial dental root where a screw part which is formed with cut surfaces is formed at an alveolar bone implant part and where a core member near the gum is formed with a continuous recessed part.

Further, Japanese Patent Publication No. H4-371146A proposes a method which enables an outermost layer constituted by an apatite-based ceramic layer to be formed reliably in a short time by forming a covering of a calcium phosphate-based compound by plasma spraying, thermal decomposition, sputtering, etc., then treating the obtained covering layer hydrothermally to convert it to an apatite-based ceramic layer, that is, recrystallized apatite covering layer.

Further, Japanese Patent Publication No. H9-99053A proposes a surface modification method which promotes stable bonding with the gum by polishing a contact surface of the surface of the core member with the gum to a mirror surface etc. so as to remove fine relief shapes, then using an aqueous solution which contains phosphate ions and calcium ions for performing hydrothermal treatment to form an oxidized coating

Furthermore, Japanese Patent Publication No. 2005-270529A propose an artificial dental root which prevents looseness which occurs due to chewing by providing at the top part of the implanted part a belt part which surrounds the same and is shaped to become tapered the further toward the bottom.

Still further, Japanese Patent Publication No. 2007-143772A proposes a dental implant which shorten the time of surgical placement of implant, enables stable placement, and provides a durable implant by providing a screw part at the bio implant part, forming cut surfaces which are parallel to the longitudinal direction at the two sides of the screw part, and giving the screw part a portion which becomes shallower in depth of groove in the upward direction.

### Citations List

### Patent Literature

PLT 1: Japanese Patent Publication No. H2-161939A
PLT 2: Japanese Patent Publication No. H4-371146A
PLT 3: Japanese Patent Publication No. H9-99053A
PLT 4: Japanese Patent Publication No. 2005-270529A
PLT 5: Japanese Patent Publication No. 2007-143772A

### Summary of Invention

### Technical Problem

However, the above such conventional artificial dental roots or dental implants still have problems to be solved. For example, the technique which is described in Japanese Patent Publication No. H4-371146A of forming a covering by plasma spraying etc. and by apatite recrystallization hydrothermal treatment is superior as a technique which gives an implant with a high bioaffinity, but plasma spraying entails melting a calcium phosphate-based compound at a superhigh temperature and spraying it on a core member, so depending on the temperature and the spraying time, even if later performing recrystallization, the crystal nuclei for the hydroxyapatite crystals would not be sufficient or the crystal growth would be insufficient. Therefore, to obtain an implant where suitably grown apatite crystals are formed on the surface, sometimes troublesome adjustment of the plasma temperature and spraying time was necessary. Further, a dental implant, as explained above, is placed while connecting the inside and outside of the body, so establishment of a stable bond at the contact surfaces of the gum and implant is an important concern.

In this regard, Japanese Patent Publication No. H9-99053A describes that stable bonding of the gum and implant is possible, but the contact surface with the gum is the boundary between the outside and inside of the body. Infection from this portion is a concern even if the bond with the alveolar bone is good since the dental implant is not stable in state, so a faster bondability with the alveolar bone is being sought. Furthermore, a dental implant also has to be able to structurally withstand the chewing force. Even if the bioaffinity is good, if fractured, the implant has to be replaced. This imposes a great burden on the patient.

Further, Japanese Patent Publication No. 2005-270529A shows a screw part which is rendered a state with uniformly relatively high screw threads at the part for placement in the alveolar bone. Further, Japanese Patent Publication No. H2-161939A proposes that a recessed part be formed at a part where the gum would enter etc. so as to increase the contact surface with the body and thereby secure better bondability with the alveolar bone or gum. However, when employing such a technique, if the load due to the chewing force etc. is applied to the implant over a long period of time, the possibility of fracture must be considered.

In this regard, by providing relief shapes at the contact portion with the biotissue, it is possible to increase the area of the contact surface and possible to secure stability by invasion into the bone structure. In these points, merits can be obtained, but the strength falls to a corresponding extent and can become a cause of fracture. Therefore, a column shaped structure which is not formed to have relief shapes (protrusions and depressions), but has a side surface which is close to a flat shape such as with a cylindrical member is sometimes preferable, but in such a case, a guide to the depth for placement in the alveolar bone becomes hard to find. Further, there is less of a contact area with the body and the stability after placement is liable to be impaired.

Furthermore, in the case of the dental implant such as proposed in Japanese Patent Publication No. 2007-143772A, the top part is formed with a support part with a volume larger than the core member of the alveolar bone implant part so that a dentist, dental assistant, etc. can process it in accordance with the state of the patient. The gum contacts the inclined part which connects this large volume and the bone implant part, so the center of gravity becomes high and the implant becomes unstable at the time of attaching the implant.

An object of the present invention is eliminate the problems in conventional dental implants (artificial dental roots) such as described above and to provide an improved dental implant which is provided with a strength able to sufficiently withstand a chewing force which is repeatedly applied over a long period of time and is provided with a fast bondability and affinity with the gum and further is excellent in extraction strength, so enables the placed implant to be securely fastened at an early stage, enables the implant to be stably used over a long period of time without fracture etc., and can contribute to a reduction in costs, and to provide a method of production of the same. Other objects of the present invention will be clear from the following detailed description.

### Solution to Problem

In view of the above problem, the inventor discovered that in a dental implant which has a recrystallized crystal surface which is obtained by growing hydroxyapatite (HAP) crystals on a contact surface with the alveolar bone, if the hydroxyapatite crystals are needle shaped and/or column shaped (for example, hexagonal column shaped) ones grown to lengths of about 2 µm to about 7 µm and form aligned crystal groups where they are aligned in a certain direction, radial crystal groups where they radially extend from the implant surface, or both aligned crystal groups and radial crystal groups which cover the implant surface, these pure hydroxyapatite crystal states give rise to a bone-forming action at portions of contact with the alveolar bone, and thereby completed the present invention.

The present invention, in one aspect, lies in a dental implant which includes a columnar core member and a covering layer which is comprised of a recrystallized apatite-based ceramic which is covered over at least part of the outer circumferential surface of the core member. In this dental implant, the covering layer which is comprised of the recrystallized apatite-based ceramic is a hydrothermal treated layer of a plasma spray-deposited covering which is formed by plasma spraying a composite which contains two or more types of calcium phosphate-based compounds which differ in melting point on an outer circumferential surface of the core member based on a melting point of a low melting point calcium phosphate-based compound in the calcium phosphate-based compounds. Note that, in working the present invention, if necessary, it is also possible to use a technique other than plasma spraying to form a covering which corresponds to the plasma spray-deposited covering and to treat the covering hydrothermally to form the targeted covering layer comprised of an apatite-based ceramic. As a suitable method for forming the covering, for example, high speed flame spraying, sputtering, etc. may be mentioned.

Further, the dental implant according to the present invention preferably further includes, in accordance with need, a hydrothermal synthesized coating which is derived from a supersaturated state aqueous solution in which phosphate ions and calcium ions are coexistent or calcium phosphate aqueous solution at the outer circumferential surface of the core member.

In the dental implant of the present invention, preferably the covering layer is comprised of length 2 µm to 7 µm needle shaped and/or column shaped crystals of the calcium phosphate-based compounds in aligned crystal groups which are comprised of the crystals formed aligned in a certain direction and/or radial crystal groups where they are formed extending from the surface toward the outer direction covering the surface.

Further, the present dental implant preferably is comprised of parts which are formed by working the core member including an alveolar bone implant part (root part) which is provided with a screw part which has cut surfaces and a dental prostheses abutment and wherein the alveolar bone implant part has the covering layer at least at the region other than the screw part.

Further, the present dental implant preferably further comprises, between the alveolar bone implant part and the dental prostheses abutment, a columnar gum contact part which is formed in a range of the core member which contacts the gum and which does not have relief shapes. This gum contact part preferably has a polished surface on its outer circumferential surface and a hydrothermal synthesized coating which is formed on this polished surface and which has a composition which contains a phosphorus component, calcium component, and combined components of the same and is the same as or different from the hydrothermal treated layer. Here, the hydrothermal synthesized coating preferably contains hydroxyapatite crystals. Further, the hydrothermal synthesized coating is a coating which is formed by hydrothermal treatment which dips the core member after formation of the plasma spray-deposited covering in a supersaturated state aqueous solution in which phosphate ions and calcium ions are coexistent or a solution in which calcium phosphate is dipped, but may also be formed by another technique.

In the dental implant of the present invention, preferably the core member which supports the covering layer has a rough surface structure which is formed in advance on its surface. That is, the surface of the core member is preferably treated to roughen it in advance.

The present invention provides, as another aspect, a method of production of a dental implant of the present invention such as explained above. In the method of the present invention, the covering layer is formed by plasma spraying a composite which contains at least two types of calcium phosphate-based compounds with different melting points on the outer circumferential surface of the core member based on the melting point of a low melting point calcium phosphate-based compound in the calcium phosphate-based compounds and hydrothermally treating the plasma spray-deposited covering which is formed by the plasma spray step to form a hydrothermal treated layer.

The method of the present invention, in accordance with need, preferably further comprises a step of forming a hydrothermal synthesized coating on the outer circumferential surface of the core member of the implant using a supersaturated state aqueous solution in which phosphate ions and calcium ions are coexistent or a calcium phosphate aqueous solution.

In working the method of production of the present invention, it is preferable to perform the plasma spraying by bringing a powder of the composite into contact with a high temperature plasma flame and spraying molten state composite on the core member and performing the hydrothermal treatment by dipping or exposing the core member which is provided with the plasma spray-deposited covering in water, an aqueous solution in which phosphate ions and calcium ions are coexistent, a calcium phosphate aqueous solution, or an atmosphere of a vapor of the same.

Further, the method preferably further comprises a core member forming step of forming an alveolar bone implant part (root part) which is provided with a screw part which has cut surfaces and a dental prostheses abutment at the core member and forms the covering layer at least at the region of the alveolar bone implant part other than the screw part.

Furthermore, the method preferably further comprises a step of forming, between the alveolar bone implant part and the dental prostheses abutment, a columnar gum contact part which does not have relief shapes in a range of the core member which contacts the gum, the step including forming a polished surface at an outer circumferential surface of the gum contact part and forming on the polished surface a hydrothermal synthesized coating which has a composition which contains a phosphorus component, calcium component, and combined components of the same and is the same as or different from the hydrothermal treated layer. Advantageous Effects of Invention

According to the present invention, as will be understood from the following detailed description, for example, by using a composite which contains two or more types of calcium phosphate-based compounds with different melting points as the covering-forming material to cover the core member by particularly plasma spraying based on the melting point of the low melting point calcium phosphate among the calcium phosphate-based compounds, then treating this hydrothermally to form a covering layer which is comprised of a recrystallized apatite-based ceramic, it is possible to promote the bone-forming action from the surface of the dental implant and promote fast stabilization with the bone tissue.

Further, regarding the contact surface with the gum, by treating the surface which is polished to remove fine relief shapes, that is, the polished surface, by hydrothermal treatment by phosphate salts and calcium salts in a supersaturated or locally supersaturated concentration so as to form a hydrothermal synthesized coating which contains hydroxyapatite crystals or their crystal nuclei, it is possible to bond with the living tissue earlier and thereby realize a stabilized dental implant.

The above advantageous effects are just examples. Other advantageous effects of the present invention will be clear from the following detailed description.

### Brief Description of the Drawings

FIG. 1 is an electron micrograph (SEM; magnification X1000) which shows the surface conditions of a recrystallized ceramic covering layer of a dental implant according to the present invention.
FIG. 2A is an electron micrograph (SEM; magnification X10000) which shows the surface conditions of the same location as photographed in FIG. 1 of the same recrystallized ceramic covering layer as that which is shown in FIG. 1.
FIG. 2B is an electron micrograph (SEM; magnification X10000) which shows the surface conditions of a portion separate from FIG. 2A of the same recrystallized ceramic covering layer as that which is shown in FIG. 1.
FIG. 3 is a schematic view which shows one preferable aspect of the dental implant according to the present invention.
FIG. 4A is a schematic view which shows another preferable aspect of the dental implant according to the present invention (aspect before formation of recrystallized ceramic covering layer and hydrothermal synthesized coating).
FIG. 4B is a schematic view which shows another preferable aspect of the dental implant according to the present invention (aspect after formation of recrystallized ceramic covering layer and hydrothermal synthesized coating).
FIG. 5 is a perspective view of the dental implant which is shown in FIG. 4B.
FIG. 6 is a schematic view which shows a state of fastening the dental implant which is shown in FIG. 4B to the alveolar bone and attaching a dental prostheses.
FIG. 7 is a graph which plots the relationship between the bone placement time period (weeks) and extraction strength (MPa) when fastening the dental implant to the alveolar bone.

### Description of Embodiments

Next, the present invention will be explained with reference to its preferred embodiments.

### [Expression of Bone-Forming Action in Pure Hydroxyapatite Crystal State]

The inventor, as explained above, discovered that if needle shaped and/or column shaped (for example, hexagonal column shaped) crystals grown to lengths of about 2 µm to about 7 µm form aligned crystal groups where they are aligned in a certain direction, radial crystal groups in states where they extend from the implant surface to the radial direction, or both aligned crystal groups and radial crystal groups which cover the implant surface, these pure hydroxyapatite crystal group states give rise to a bone-forming action at contact portions with the alveolar bone.

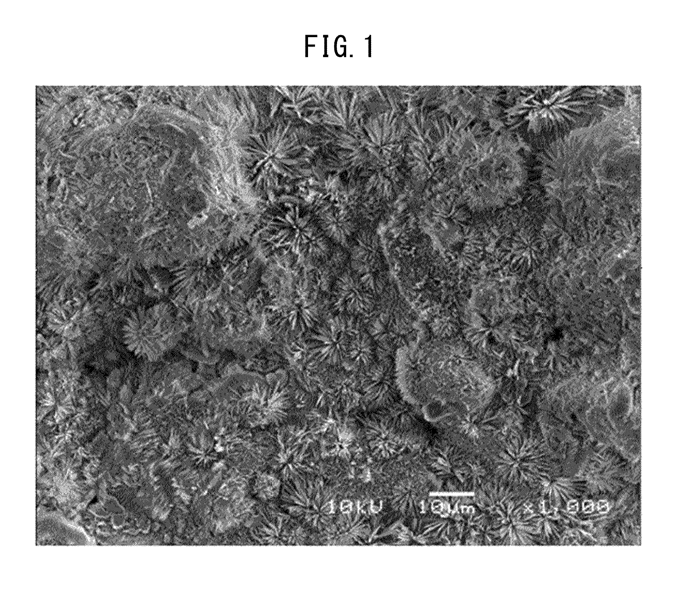

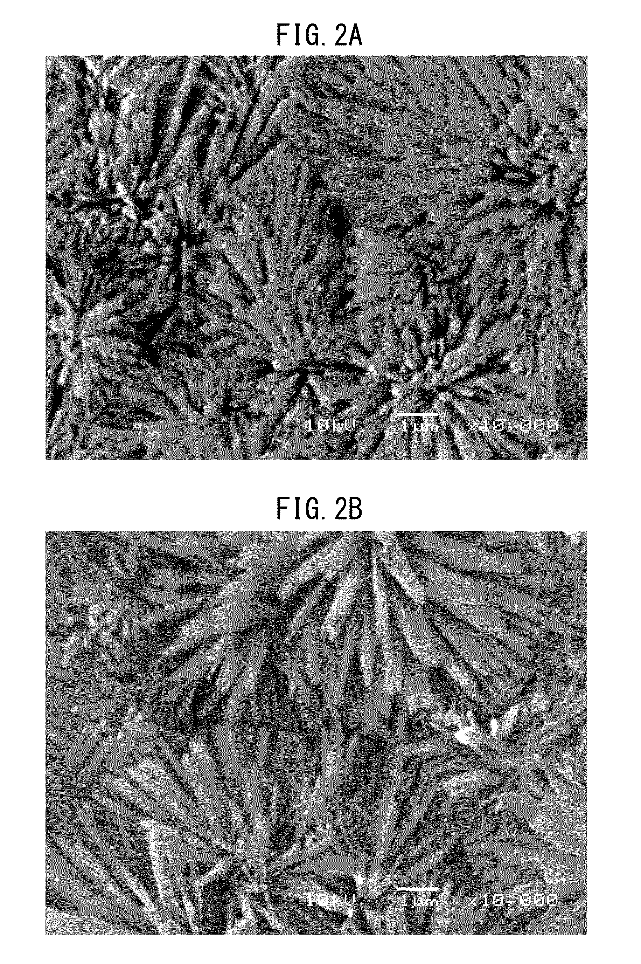

For sufficient understanding of the present invention, referring to FIG. 1 and to FIG. 2A and FIG. 2B, first, the aligned crystal groups and radial crystal groups will be explained. FIG. 1 is a scan type electron micrograph (SEM; magnification X1000) which shows the surface conditions of a recrystallized ceramic covering layer of a dental implant according to the present invention. Further, FIG. 2A and FIG. 2B photograph the same recrystallized ceramic covering layer as that which is shown in FIG. 1 but increased in magnification. That is, FIG. 2A is an SEM (magnification X10000) which shows the surface conditions at a portion the same as that photographed in FIG. 1 of the same recrystallized ceramic covering layer as that which is shown in FIG. 1. Further, FIG. 2B is an SEM (magnification X10000) which shows the surface conditions at a portion different from FIG. 2A of the same recrystallized ceramic covering layer as that which is shown in FIG. 1.

When used in the present invention, "aligned crystal groups" indicate arrays of nearby crystals in states aligned (oriented) in a certain direction as shown in for example FIG. 2A and FIG. 2B. Further, "radial crystal groups" indicate bunches of nearby crystals in states extending radially from the surface of the dental implant toward the outer direction. Accordingly, FIG. 2A and FIG. 2B show the state where a plurality of aligned crystal groups and a plurality of radial crystal groups cover the implant surface. More specifically, in FIG. 2A, numerous hexagonal column shaped hydroxyapatite (HAP) crystals are photographed, while in FIG. 2B, numerous needle shaped hydroxyapatite crystals are photographed.

### [Formation of Implant Surface Covered by Hydroxyapatite Crystal States]

As explained above, to establish a state where a plurality of aligned crystal groups and a plurality of radial crystal groups cover the implant surface, according to the present invention, for example the following may be performed to form an implant surface which is covered by specific hydroxyapatite crystal states.

A material, called a "composite" in the present invention, including two or more types of calcium phosphate-based compounds with different melting points is prepared as the starting material. Next, when forming a recrystallized ceramic covering layer from this material, while it is also possible to use another covering-forming method if necessary so long as it is possible to form a comparable satisfactory recrystallized ceramic covering layer, preferably plasma spraying is used. Plasma spraying basically can be performed by the process and conditions which are usually employed in this technical field and may be freely changed in accordance with need. However, when performing plasma spraying in accordance with the present invention, it is necessary to perform the plasma spraying based on the melting point temperature of the low melting point calcium phosphate-based compound among the plurality of calcium phosphate-based compounds. By plasma spraying in this way, it is possible to obtain, as the spray-deposited covering which is formed on the core member (base member) which forms the main body of the implant, a covering layer with sufficient bonding with the core member while containing a large number of internal crystal nuclei for recrystallization. In the case of this covering layer, a specific crystal structure can be realized in which the crystal state after recrystallization becomes needle and/or columnar crystals of calcium phosphate-based compounds, the lengths become about 2 µm to about 7 µm, and aligned crystal groups where the crystals are aligned in the same direction and/or radial crystal groups cover the covering layer. As a result of such a specific crystal structure being realized, according to the present invention, surprisingly, the bone-forming action arising derived from the implant surface enables the placed implant to be stabilized quickly in the alveolar bone.

In working the present invention, as the core member, any metal material or ceramic material which is usually used for fabricating a dental implant may be used alone or else two or more types of materials may be combined and used as a composite. As suitable materials, for example, pure titanium, a titanium-nickel alloy or other titanium alloy, tantalum, stainless steel, zirconia, partially stabilized zirconia, corundum and other manmade sapphire, alumina (aluminum oxide), CaO-Na₂O-P₂O₅-SiO₂ type glass (bioglass) and other metal materials or ceramic materials and composites of these materials may be mentioned. Among these materials, pure titanium or titanium alloy is preferable in terms of strength, bioaffinity, etc.

Further, the composite serving as the starting material for forming the recrystallized ceramic covering layer can be prepared by combining various calcium phosphate-based compounds with different melting points. As suitable calcium phosphate-based compounds, for example, hydroxyapatite, tetracalcium phosphate, α-tricalcium phosphate, β-tricalcium phosphate, dicalcium phosphate, dicalcium phosphate dihydrate, etc. may be mentioned. These calcium phosphate-based compounds are usually used in combinations of two types, but may also be combinations of three or more types in accordance with need. Note that, when selecting the calcium phosphate-based compounds, so long as the hydroxyapatite recrystallizes after hydrothermal treatment and a state is established where the implant surface becomes comprised of a plurality of aligned crystal groups and a plurality of radial crystal groups, it is also possible to use something other than the above-mentioned calcium phosphate-based compounds.

In the present invention, as explained above, to form the recrystallized ceramic covering layer, after using plasma spraying to form the covering layer, it is necessary to subject the covering layer to hydrothermal treatment. The hydrothermal treatment, like plasma spraying, can basically be performed by the technique and under the conditions which are usually employed in this technical field and can be freely changed in accordance with need. Hydrothermal treatment usually means heat treatment of a core member after formation of a plasma spray-deposited covering at a high pressure in for example water or a water-based atmosphere such as a water vapor atmosphere. For example, it is possible to perform the hydrothermal treatment by dipping the core member in water at a high temperature of about 90°C or more, preferably about 110 to 125°C, under a high pressure of about 0.1 to 0.2 MPa for treatment for about 1 to 200 hours. Further, for example, instead of dipping in water, the core member can be exposed to a water vapor atmosphere.

### [Bone-Forming Action From Implant Surface]

In the present invention, the recrystallized hydroxyapatite crystals which are formed at the portion which contacts the bone tissue are needle shaped and/or for example hexagonal column or other column shaped in form and are aligned grown in a certain direction or arranged radially. This needle shaped and/or column shaped form becomes a force for securing epitaxy with the collagen of the bone tissue and can given rise to a bone-forming action.

Specifically, as shown in FIG. 1 and FIG. 2A and FIG. 2B, at the surface where regions of needle shaped and/or column shaped pure recrystallized hydroxyapatite aligned grown in a certain direction and regions of the same formed radially densely congregate, osteoclasts which are separated from blood components interpret the needle shaped and/or column shaped recrystallized hydroxyapatite as relief shapes of natural bone, stick to the tips of the crystals, and dissolve and absorb the crystals from their tips.

Next, after the osteoclasts dissolve and absorb the hydroxyapatite crystals, the osteoblasts start to become active based on a signal or state due to the osteoclasts. The osteoblasts enter into the gaps between hydroxyapatite crystals centered at the portions where the osteoclasts dissolve and absorb the needle shaped and/or column shaped hydroxyapatite crystals, in some cases while deforming etc., where they become active. Here, the osteopontin, osteocalcin, bone sialoprotein, and other bone-related proteins which osteoblasts etc. secrete and the collagen having a fibrous shape etc. which osteoblasts etc. produce are adsorbed at the gaps between needle shaped and/or column shaped hydroxyapatite crystals and stored there.

Furthermore, hydroxyapatite molecules deposit on the surfaces of the collagen fibers. Part of the hydroxyapatite molecules which deposit on the surfaces of the collagen fibers form supersaturated "places" and thereby cause fast precipitation of hydroxyapatite crystals in the body. The deposited and precipitated hydroxyapatite cause collagen fibers to bond with each other or cause newly formed crystal apatite and needle shaped and/or column shaped hydroxyapatite crystals to bond or otherwise perform a cement-like role to form the foundation for bone. Furthermore, the osteoblasts after forming the new bone become bone cells which are embedded in this foundation while the above action is repeated and as a result the action of forming new bone advances.

In this way, an implant surface which is formed by needle shaped and/or column shaped (for example, hexagonal column) hydroxyapatite crystals supports the action of osteoblasts etc. and forms excellent starting points for forming new bone. The bone-forming action from this implant surface is performed together with the bone-forming action from the alveolar bone side, so it is possible to quickly realize a strong bond between the implant surface and bone tissue.

### [Comparison With Conventional Dental Implant Not Having Recrystallized Ceramic Covering Layer]

In this regard, in the case of a conventional dental implant where the dental implant is comprised of only a core member and there is no recrystallized ceramic covering layer at the outer circumferential surface of the core member, the above-mentioned such bone-forming action cannot be expected. This will be explained with reference to the example of the typical case of using a titanium member as the core member.

Usually, with an implant of only a titanium core member, since titanium itself is not bioactive, new bone cannot grow from the implant body side. Further, since there is no bone-forming action, in the case of a titanium core member, only the increase in new bone from the alveolar bone side promotes contact with the implant. Further, the implant and the bone tissue in the end only contact each other. There is no chemical action other than physical contact between the implant and the bone tissue.

As opposed to this, in the present invention, as explained above, by inducing osteoclasts, as a result osteoblasts are attracted and a bone forming action comes into play and new bone is formed. Further, by new bone being formed, a chemical bond is obtained by ion exchange between the implant and alveolar bone. Therefore, due to the bone-forming actions of both the bone and implant, faster bonding of the bone can be achieved compared with an implant of only a titanium core member.

Further, in the case of the present invention, the implant surface is formed by regions where the needle shaped and/or column shaped crystals of the calcium phosphate-based compound are aligned in the same direction (aligned crystal group) and regions where they are arranged radially from the implant surface to the outer direction (radial crystal group), so the new bone parts which are formed at these regions closely bond with each other, so a higher bonding strength can be obtained.

### [Formation of Recrystallized Ceramic Covering Layer]

According to the present invention, the recrystallized ceramic covering layer can be formed by plasma spraying using a plurality of calcium phosphate-based compounds with different melting points as a composite. Here, "a plurality of calcium phosphate-based compounds with different melting points" means, for example, a combination of high melting point tricalcium phosphate (MP: 1670°C or more) and low melting point hydroxyapatite (MP: 1650°C or less) or another possible combination. Further, "plasma spraying based on the melting point of the low melting point calcium phosphate-based compound" means setting the reference when performing plasma spraying to the low melting point calcium phosphate-based compound and adjusting the spraying conditions at the time of plasma spraying (for example, the type and flow rate of the carrier gas, plasma gas, secondary gas, and other various types of gases, pressure, temperature, voltage, and current, frequency, etc. for plasma output, for example, arc discharge) to perform plasma spraying under spraying conditions which are set to a heat content whereby the powder of the low melting point calcium phosphate-based compound will substantially melt. Note that, when performing plasma spraying in this way, due to the need to leave behind aligned crystal groups or radial crystal groups, it is necessary to apply conditions resulting in a state where crystal powder remains in the powder of the starting material comprised of the covered high melting point calcium phosphate-based compound. Note that, the above-mentioned "melting point (MP)" is a representative value. In actuality, the difference in the melting points of the two is sometimes 100°C or more.

Further, as another method which can be applied to the present invention, the method of setting the temperature at the time of plasma spraying to a temperature the same as or higher than the melting point of the high melting point calcium phosphate-based compound while shortening the spraying time so as to melt the low melting point calcium phosphate-based compound, then performing the spraying until a timing before the high melting point calcium phosphate-based compound completely becomes a molten state and up to when a large amount of crystals of the powder of the starting material remains may be illustrated.

That is, according to another method, it is also possible to employ a plasma spraying method wherein plasma spraying is performed to spray a low melting point calcium phosphate-based compound and form a covering layer with a high bondability, then the conditions are adjusted so that a state is formed where the covering layer contains a high melting point calcium phosphate-based compound in a state with crystals of the spraying material powder remaining in it. Note that, the low melting point calcium phosphate-based compound usually preferably is mixed in by a ratio of 1 to 15% or so with respect to the total weight of the calcium phosphate-based compound.

When performing the plasma spraying, the plasma temperature may be adjusted by, for example, adjusting the current and voltage and the frequency when performing the plasma discharge and the amounts, types, pressures, etc. of the various types of gases or by adjusting the amounts and components of the alkali metals mixed in to utilize hot plasma adjusted in degree of ionization. Further, even when using a method other than plasma spraying (for example, high speed flame spraying) to form the spray covering, it is possible to expect that similar advantageous effects to plasma spraying will be obtained by adjusting the heat source.

Further, the high melting point calcium phosphate-based compound need only be one whereby hydrothermal treatment causes the growth of needle shaped and/or column shaped crystals of hydroxyapatite, orients them on the implant surface which contacts the alveolar bone, and forms aligned crystal groups and/or radial crystal groups. As this calcium phosphate-based compound, preferably, α-tricalcium phosphate and/or β-tricalcium phosphate may be illustrated.

### [Formation of Aligned Crystal Groups and/or Radial Crystal Groups by Hydrothermal Treatment]

Hydrothermal treatment, as explained briefly previously as well, means treatment by dipping or exposing the core member having a spray-deposited covering in water, an aqueous solution in which phosphate ions and calcium ions are coexistent or an aqueous solution of a calcium phosphate (coexistent compound) or an atmosphere of a vapor of the same and continuing that state as is under a high temperature and high pressure to form a hydrothermal synthesized coating. The hydrothermal treatment is preferably performed using a device for autoclaving.

When performing hydrothermal treatment, the phosphate ions to be made coexistent in the water are represented by PO₄ and, for example, can be contained in not only hydroxyapatite, tricalcium phosphate, tetracalcium phosphate, calcium hydrogenphosphate, and other calcium phosphate-based compounds, but also sodium dihydrogenphosphate, disodium hydrogenphosphate, dicalcium hydrogenphosphate, ammonium phosphate, and other compounds. Further, the calcium ions can be contained in not only the above calcium phosphate compounds, but also calcium carbonate, calcium chloride, calcium nitrate, calcium hydroxide, etc. Further, considering the corrosion resistance, chemical stability, etc. of metal, ones to which Mg, Sr, Fe, Cr, Ti, Zr, Co, Mo, Al, Si, V, F, and other various types of ions are added may also be encompassed by coexistent compounds.

Further, to promote crystallization and crystal growth, the concentrations of phosphate ions and calcium ions of the aqueous solution and water-based atmosphere to be dipped in or exposed to are preferably made supersaturated or locally supersaturated states. The supersaturated state may be formed by the means of administering calcium phosphate etc. to the aqueous solution to an extent whereby the concentrations of phosphate ions and calcium ions become supersaturated as an example, but in addition, when dipping the core member after plasma spraying in water, an amount of water whereby the covered molten state low melting point calcium phosphate-based compound forms a supersaturated state aqueous solution by the amount of dissolution is preferable. Further, it is also possible to add the residue of calcium phosphate-based composite which escapes from the core member at the time of plasma spraying.

The treatment time of the hydrothermal treatment is usually about 1 to 200 hours, but is preferably about 5 to 50 hours, more preferably about 7 to 28 hours. The treatment time should be suitably adjusted in accordance with the number of the core members to be treated, the thickness and material of the covering layer, and other factors and is not limited to the above range. Due to the hydrothermal treatment, the crystal nuclei of the covering layer which remain or are produced grow and needle shaped and/or column shaped (preferably hexagonal column shaped) hydroxyapatite is formed.

When crystal state hydroxyapatite is formed, the length of the crystals in the crystal state of hydroxyapatite is preferably about 2 to 7 µm. A state where the aligned crystal groups of crystals aligned so as to follow along the surface of the core member and the radial crystal groups of crystals which are formed radially from the surface of the core member are coexistent and comprise the contact surface of the implant with the alveolar bone is preferable. Note that, the surface of the implant which contacts the alveolar bone is preferably treated in advance by sandblasting etc. to roughen the surface, so the direction of alignment sometimes follows along the relief shapes at the surface. An aligned state and radial state of crystals such as shown in FIG. 1 and FIG. 2A and FIG. 2B are sufficient.

Here, if the lengths of the grown crystals are less than 2 µm, calcium phosphate-based compounds other than hydroxyapatite may remain. Further, if over 7 µm, the possibility of the crystals breaking becomes higher. In this way, in the crystal growth by hydrothermal treatment, the time of the hydrothermal treatment is illustrated as being 7 hours to 28 hours or so, but depending on the additives at the time of hydrothermal treatment or the state of the covering layer, sometimes may also be outside the range of 7 to 28 hours.

The temperature of the hydrothermal treatment is preferably usually about 110 to 125°C. If under 110°C, the crystal growth is insufficient, while conversely if over 125°C, the covering layer becomes fragile. Regarding the time of the above-mentioned hydrothermal treatment as well, reasons similar to the above can be applied for outside that range. Note that, in the growth of crystals by hydrothermal treatment, for example, if making the treatment time longer, the crystals grow more, but if exceeding the above range of length of crystals, the crystals have grown too much and become fragile and peeling occurs.

### [Formation of Hydrothermal Synthesized Coating at Gum Contact Part]

According to the present invention, when the dental implant is comprised of a main body (core member) provided with an alveolar bone implant part and dental prostheses abutment, it is preferably further provided with a column shaped gum contact part between the alveolar bone implant part and dental prostheses abutment. Here, the column shaped gum contact part should be formed in a range contacting the gum at the outer circumferential surface of the core member, so preferably has no roughness (protrusions and depressions) at its surface. That is, the gum contact part is preferably provided with a polished surface which is formed at the outer circumferential surface and a hydrothermal synthesized coating which is formed on this polished surface. The hydrothermal synthesized coating can include a phosphorus component, calcium component, and combined components of the same. Its composition may be the same composition as a hydrothermal treated layer which is formed by the above-mentioned hydrothermal treatment. Otherwise, it may be a different composition derived from being formed by other hydrothermal treatment.

The gum contact part preferably can be formed by polishing the contact surface of a metal core member of a shape closer to a cylindrical member with the gum by preferably polishing it to a mirror surface and forming on the polished surface which is formed a hydrothermal synthesized coating which contains a calcium component and phosphorus component and a hydroxyapatite or other calcium phosphate-based compound which is preferably crystallized or is formed with crystals which are grown due to contact with saliva or other moisture after placement. The core member in this case may, in accordance with need, be formed from a material other than metal, for example, a ceramic material etc. The mirror polishing or other polishing can be performed in accordance with ordinary methods. The hydrothermal treatment for forming the hydrothermal synthesized coating can be performed as explained above. By forming a hydrothermal synthesized coating at the outer circumferential surface of the core member which contacts the gum, it is possible to improve the affinity between the core member and the gum or fast bondability by growth of hydroxyapatite crystals after placement so as to quickly close the opening between the inside and outside of the body at the gum contact portion and realize a dental implant which is stable over a long period of time.

The hydrothermal synthesized coating, as will be understood from the above, can be formed at any stage when fabricating the dental implant, but it is preferable to form it simultaneously with the time of hydrothermal treatment when spraying a calcium phosphate composite by plasma spraying, then forming and growing hydroxyapatite crystals.

The hydrothermal synthesized coating is preferably formed in a state where crystal nuclei are present or a state in which they will be formed by hydrothermal treatment where the polished surface of the core member is dipped in a supersaturated or locally supersaturated aqueous solution. Further, the aqueous solution which is used need not be a supersaturated aqueous solution and may be also be an aqueous solution of a concentration close to supersaturation. Alternatively, if lengthening the hydrothermal synthesis treatment time, sometimes it is also possible to omit the use of a supersaturated aqueous solution.

Furthermore, in the state where the hydrothermal synthesized coating contacts the gum, contact with saliva and other bodily fluids causes phosphate ions and calcium ions to dissolve out from the hydrothermal synthesized coating. An aqueous solution of these coexistent ions becomes locally present in the supersaturated or locally supersaturated state whereby these crystal nuclei grow and form hydroxyapatite crystals. In such a case, the bondability between the core member and gum will be improved more.

### [Shape of Core Member]

The core member is preferably used in a shape closer to a cylindrical member. The portion of a "shape closer to a cylindrical member" is preferably formed by at least a tubular member with no relief shapes from the screw part (alveolar bone implant part) to the gum contact part of the core member.

Furthermore in the present invention, the alveolar bone implant part comprised of the column shaped main body forms a screw part with threads of a height equal to or less than the radius of the column shape and with grooves of a depth which becomes shallower the more toward the support direction and which finally becomes the same as the surface of the column shape and forms a dental prostheses (crown) abutment which is provided with a shape converging in the support direction. Further, at the alveolar bone implant part comprised of the column shaped main body, the portion which can be involved in the placement is formed with the recrystallized ceramic covering layer. Furthermore, the gum contact part also comprised of the column shaped main body can be polished to a mirror surface to form a polished surface and be made closer in shape to a column shaped member thereby greatly avoiding the possibility of fracture.

Further, the dental implant of the present invention has the main body shaped close to a cylindrical member. Due to this, it becomes featureless when viewed. Up to where the alveolar bone implant part extends has to be carefully considered, but concern is pointless. The reason why is that, according to the present invention, the boundary between the recrystallized ceramic covering layer and the mirror surface gum contact part can be clearly set as the position for placement in the alveolar bone, so at the time of surgical placement of the implant, the user can easily suitably place the implant.

### [Hydrothermal Synthesized Coating Formed at Contact Surface of Core Member and Gum]

In the present invention, as explained above, a hydrothermal synthesized coating is formed at the contact surface of the alveolar bone implant part of the core member and the gum.

A metal material first of all and other material which is used as the core member often has fine relief shapes (protrusions and depressions) at the surface. Therefore, to remove the relief shapes, it is recommended to polish the core member in advance using for example mechanical polishing, chemical polishing, electrolytic polishing, or other polishing methods which use sandpaper, surface buffering, roller burnishing, or other polishing means. Among the polishing methods, polishing to a mirror surface is preferable. Preferably, after polishing to a mirror surface, the core member is treated by hydrothermal synthesis treatment to thereby form a hydrothermal synthesized coating which contains a phosphorus component and calcium component or these two components.

The hydrothermal synthesis treatment is preferably performed at the same timing as the hydrothermal treatment for recrystallization which forms and grows hydroxyapatite crystals at the covering layer formed by the above-mentioned calcium phosphate-based composite. Accordingly, hydrothermal synthesis treatment sometimes is abbreviated as "hydrothermal treatment". The treatment time of hydrothermal synthesis treatment may be the same as the treatment time of the hydrothermal treatment of the spray-deposited covering layer surface or may be less or more, but from the viewpoint of making the production process the same, the hydrothermal treatment and the hydrothermal synthesis treatment are preferably performed by the same treatment time and same treatment content.

Examples of the thickness and components of hydrothermal synthesized coatings which are formed by hydrothermal synthesis treatment including a phosphorus component and calcium component after polishing to a mirror surface are shown in the following Table 1.

**Table 1**

| | Temperature (%) | | | | Thickness of coating (nm) |
|---|---|---|---|---|---|
| | Ti | O | Ca | P | |
| Untreated | 27.0 | 73.0 | - | - | 4 |
| Treated | 26.6 | 72.2 | 3.2 | 1.1 | 8 |

In the above Table 1, after treatment, the calcium component and phosphorus component are respectively present. The thickness of the coating including the calcium phosphate-based compound due to the hydrothermal synthesis becomes double. In this way, the metal material or other core member surface is formed with a hydrothermal synthesized coating which contains a phosphorus component and calcium component and has about double the thickness, so in the dental implant of the present invention, fast bonding of the hydrothermal synthesized coating and gum can be achieved.

Further, the gum and oral cavity are portions rich in moisture due to saliva and other bodily fluids, so the hydrothermal synthesized coating which is formed at this gum part according to the present invention enables the precipitation and growth of hydroxyapatite crystals even after placement of the implant and can therefore promote bioaffinity and gum bondability.

If shown for reference in hydrothermal synthesis treatment, the formation of a multilayer crystal film on a titanium core member by the technique of hydrothermal synthesis treatment can be deemed to be explained in the following reference and homepage: J. Ceram. Soc. Japan, Vol. 107, No. 10, pp. 907-912, 1999 and http://eim.ceram.titech.ac.jp/fields_j/hydrothermal/multi .html.

A titanium core member or other core member is preferably polished, preferably is polished to a mirror surface or is polished to a state close to this, so as to remove fine relief shapes of the surface, then is supplied in that state to the step of forming the hydrothermal synthesized coating. According to this technique, it is possible to form a hydrothermal synthesized coating which contains hydroxyapatite crystals or crystal nuclei in a state polished to remove fine relief shapes at the contact surface of the core member with the gum, so fast bondability between the core member and gum can be realized.

Above, the dental implant and method of production of the same according to the present invention were clarified through an explanation of particularly
(1) the expression of a bone-forming action in pure hydroxyapatite crystal states,
(2) the formation of an implant surface covered by hydroxyapatite crystal states,
(3) the bone-forming action from the implant surface,
(4) comparison with a conventional dental implant which does not have a recrystallized ceramic covering layer,
(5) the formation of the recrystallized ceramic covering layer,
(6) the formation of aligned crystal groups and/or radial crystal groups by hydrothermal treatment,
(7) the formation of a hydrothermal synthesized coating at the gum contact part,
(8) the shape of the core member, and
(9) the hydrothermal synthesized coating which is formed at the contact surface of the core member and gum.
Next, preferred embodiments of the present invention will be further explained.

According to the present invention, preferably, a composite which contains calcium phosphate-based compounds of different melting points is used as the starting material for plasma spraying based on the melting point temperature of the low melting point calcium phosphate-based compound among them on a core member which is sandblasted in advance so as to form a so-called "recrystallized ceramic covering layer". In the present invention, this recrystallized ceramic covering layer is in particular covered over the portion of the implant main body to be placed in the alveolar bone to thereby enable a large number of crystal nuclei based on the high melting point calcium phosphate-based compound to remain at the covering layer surface. As a result, after crystal growth by the subsequently performed hydrothermal treatment, at least at the surface of the core member which is placed in the bone tissue, it is possible to form a recrystallized ceramic covering layer in a state covered over its surface by needle shaped and/or column shaped crystals of calcium phosphate-based compounds of lengths of about 2 to 7 µm in arrays of crystals (crystal groups) aligned in the same direction and/or radial crystal groups.

In the present invention, as explained above, by forming on the outer circumferential surface of the core member a covering layer which is covered by regions of needle shaped and/or column shaped crystallized hydroxyapatite aligned in the same direction and/or regions where they extend radially from the implant surface toward the outer direction, it is possible to realize a more effective bone-forming action at the covering layer side and, accordingly, obtain an implant which is stably fastened in a short period of time after placing the implant in the alveolar bone.

On the other hand, regarding the contact surface of the core member with the gum, that is, the gum contact part, it is possible to promote bonding with the gum by adopting a configuration forming on the contact surface with the gum a polished surface which is polished to an extent whereby the surface relief is minimized and a hydrothermal synthesized coating which contains crystals and/or crystal nuclei of calcium phosphate-based compounds. Further, it is possible to establish a sufficient bond between the core member and the gum, so it is possible to prevent infection by bacteria between the bonded surfaces of the implant and the gum which is caused by rocking motion due to chewing on the dental implant which runs between the inside and outside of the body and to realize fast biostabilization.

Further, when forming the spray-deposited covering layer comprised of the calcium phosphate-based composite at the contact surface of the alveolar bone and core member plus providing the hydrothermal synthesized coating at the contact surface of the gum and the core member, it is preferable to roughen the alveolar bone implant part in the core member (for example, sandblast the surface to form relief shapes), then form the spray-deposited covering layer and form a polished surface with no surface relief at the gum contact surface, then perform hydrothermal treatment to form the hydrothermal synthesized coating. Note that, the spray-deposited covering layer and the hydrothermal synthesized coating are preferably both formed on a single core member, but, for example, in the case of a two-piece type where the alveolar bone implant part and the gum contact part are separate, treatment for the respective objects may be performed.

Furthermore, the present invention preferably reduces the danger of fracture etc. to a minimum even if a chewing force or other large load is applied by forming the screw part at one end of the column shaped core member to just the necessary amount, making the contact portion of the range of placement in the bone tissue and the gum cylindrical at other portions, and furthermore forming the above-mentioned recrystallized ceramic covering layer at the portion which is placed inside the bone tissue and forming a polished surface and a hydrothermal synthesized coating which contains crystals and/or crystal nuclei of calcium phosphate compounds at the contact part with the gum. By configuring the core member in this way, at the contact surface with the alveolar bone, the bone-forming action from the implant surface causes fast bonding with the alveolar bone and enables fast bonding with the gum and bone tissue, so placement of a dental implant which will be stable over a long period of time becomes possible.

Furthermore, the present invention need only be a screw part where the groove of the thread becomes shallower toward the support direction of the core member and finally becomes equal to the surface of the main body of the core member so as to enable chewing to be withstood over a long period of time and a cylindrical member with a range of placement in the alveolar bone set by the portions of the recrystallized ceramic covering layer and hydrothermal synthesized coating, but depending on the case, the implant may also be formed by a square column, triangular column, design mimicking a dental root shape, etc.

The present invention can form a surface from which fine relief shapes of the surface are removed at the gum constant surface where the inside and outside of the body are connected and can form a hydrothermal synthesized coating which contains crystals and/or crystal nuclei of calcium phosphate-based compounds at that surface so as to quickly prevent infection from the contact surfaces of the gum and implant, so stable use as a dental implant becomes possible even if a shape having a configuration close to a cylindrical shape.

Such a hydrothermal synthesized coating, at the time of hydrothermal treatment, uses water or a calcium phosphate aqueous solution, or an aqueous solution in which phosphate ions and calcium ions are coexistent and in which the concentration of phosphoric acid etc. becomes supersaturated or locally supersaturated. Due to this, the plasma spray-deposited covering layer can promote the growth of hydroxyapatite crystals and can form hydroxyapatite crystals at the contact surface with the gum.

Note that, when dipping the core member in water, the melt which is formed when performing plasma spraying based on the melting point of the low melting point calcium phosphate-based compound in the composite of different melting point calcium phosphate-based compounds dissolves into the water, so it is possible to prepare a supersaturated or locally supersaturated calcium phosphate aqueous solution by reducing the amount of water.

The above prepared supersaturated or locally supersaturated calcium phosphate aqueous solution can cause growth of the crystal nuclei which are present in the covering of calcium phosphate-based compound which was formed by plasma spraying, form regions of needle shaped and/or column shaped hydroxyapatite crystals aligned in the same direction and regions of the same extending radially from the implant surface toward the outer direction, and form a hydrothermal synthesized coating where hydroxyapatite crystals can be formed at the polished surface of the contact surface with the gum.

Further, by hydrothermally treating a single implant core member which has a gum contact surface which is polished to render it a state with no fine relief shapes at the surface and an alveolar bone placement part which is sandblasted etc. to roughen its surface, then is covered with calcium phosphate-based compounds by plasma spraying, the alveolar bone contact surface is formed with regions of needle shaped and/or column shaped hydroxyapatite crystals aligned in the same direction and/or regions of crystals extending radially from the implant surface toward the outer direction, and the hydrothermal synthesized coating at the contract surface with the gum is formed with hydroxyapatite crystals and/or crystal nuclei.

### Examples

Next, the present invention will be explained with reference to the examples. Note that, the present invention is not limited by these examples.

### Dental Implant 1

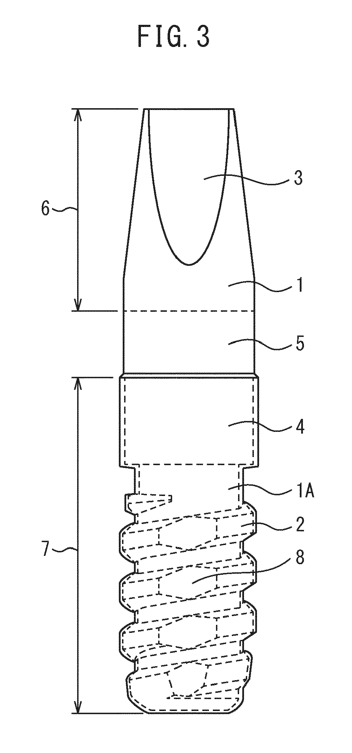

One example of the dental implant according to the present invention will be explained with reference to FIG. 3 which shows one preferable aspect of the implant.

In the dental implant (artificial dental root) which is shown in FIG. 3, reference notation 1 is the main body (core member) and is formed into a circular column shape from a titanium material, titanium alloy material, etc. The bottom part of the main body 1 is an alveolar bone implant part (root part) and is provided with a screw part 2. The screw part 2 is used when inserting and screwing an implant in a hole which is drilled in the alveolar bone. The screw part 2 is provided with cut surfaces 8 which act to prevent rotation of the main body 1. The cut surfaces 8 are preferably provided at facing surfaces (not shown). Further, at the top part of the screw part 2, a recessed part 1A is formed.

The top part of the main body 1 is provided with a dental prostheses abutment 3. The dental prostheses abutment 3 is the part for attachment of the dental prostheses (crown) when the implant is a one-piece type. In the case of a two-piece type, it is the part forming the top structure and, for example, can have a shape converging toward the top direction of the main body 1.

The main body 1, as illustrated, has a recrystallized ceramic covering layer 4 at its outer circumferential surface. The recrystallized ceramic covering layer 4 can in some cases also be called a recrystallized ceramic covering and is formed by a recrystallized ceramic covering which is comprised of recrystallized hydroxyapatite or other calcium phosphate. The covering layer 4 is in a state with its surface roughened by recrystallization and is covered over the outer circumferential surface of the main body 1 to an extent of a state one size larger than the diameter of the main body 1.

The main body 1 further sometimes has a hydrothermal synthesized coating 5 which can be called a "hydrothermal synthesized coating". The hydrothermal synthesized coating 5 is a part at the surface of the main body which is buffed or otherwise treated by a polishing material to render it preferably a mirror surface in state and mainly contacts the gum. Note that, this part need not be finished to a mirror surface. It sometimes need only be polished to a state resulting in a low reactivity with the gum.

The hydrothermal synthesized coating 5 can be formed by performing hydrothermal synthesis treatment by an aqueous solution of tricalcium phosphate or other phosphate salt and calcium salt preferably rendered a supersaturated state. The hydrothermal synthesized coating 5 is provided with a predetermined thickness and contains a phosphorus component and calcium component. Depending on the case, it also contains hydroxyapatite crystals and/or crystal nuclei. Note that, the hydrothermal synthesized coating 5 is thicker than the thickness of the oxidized coating which is naturally formed when the surface is not treated, but even in a state where hydroxyapatite crystals and/or crystal nuclei are formed, it is thin and transparent, so the brightness of the mirror surface which is present as its foundation is not impaired. It is possible to view this brightness to adjust the depth of the implant when placing it in the alveolar bone. Further, the hydrothermal synthesized coating 5 is formed by performing the above-mentioned hydrothermal synthesis treatment, so a phosphorus component and calcium component are contained. Furthermore, in many instances, these components combine to be converted into a hydroxyapatite or other calcium phosphate-based compound layer or crystals are formed. Furthermore, contact with saliva or other moisture can promote crystallization and promote bonding with the gum.

The main body 1, when observed as sections in the longitudinal direction, is comprised of a dental prostheses, dental prostheses abutment 6 which is formed by the part attaching the crown (corresponding to above-mentioned dental prostheses abutment 3), gum contact part 5 below that which has the same range as the above-mentioned hydrothermal synthesized coating, and alveolar bone implant part 7 which has a range the same as the above-mentioned recrystallized ceramic covering layer 4. Further, the portions of the hydrothermal synthesized coating 5 and the recrystallized ceramic covering layer 4 preferably form cylindrical shapes with little relief shapes in state so as to improve the durability.

### Dental Implant 2

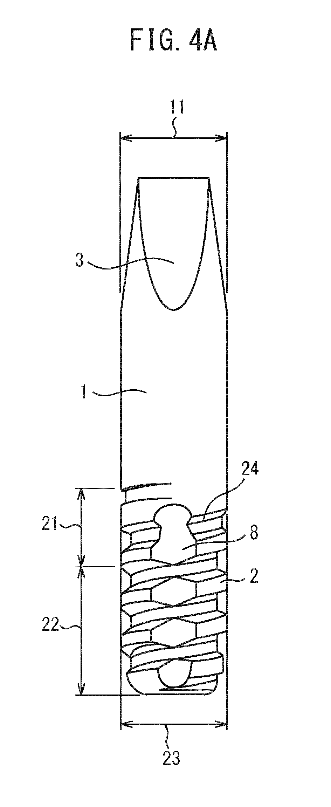

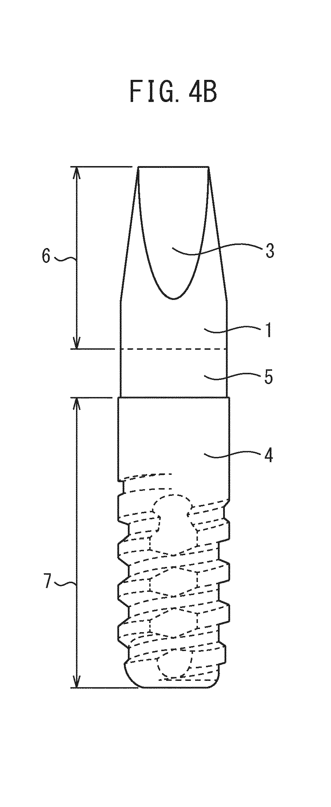

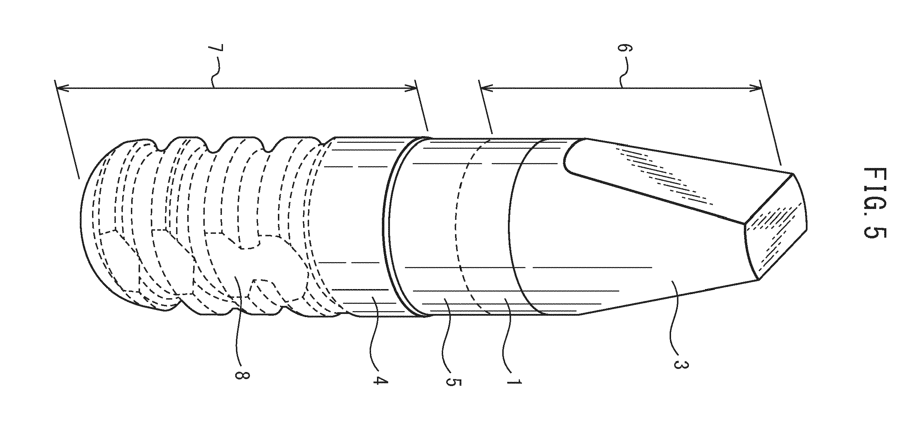

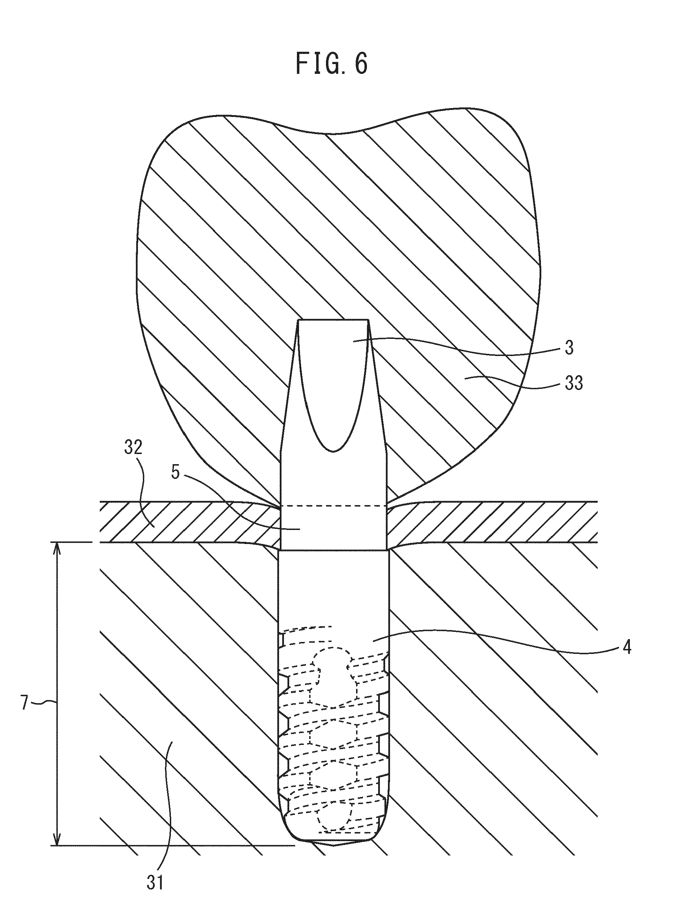

Another example of the dental implant according to the present invention will be explained with reference to FIG. 4A, FIG. 4B, and FIG. 5 which show one preferable aspect of the implant and FIG. 6. Note that, FIG. 4A is a schematic view which shows the state in the dental implant before forming the recrystallized ceramic covering layer and hydrothermal synthesized coating on it while FIG. 4B is a schematic view which shows the state in the same implant after forming the recrystallized ceramic covering layer and hydrothermal synthesized coating on it, while further FIG. 5 is a perspective view of the implant which is shown in FIG. 4B. In this example, the recessed part 1A between the screw part 2 and the main body 1 which is shown in FIG. 3 is omitted. This part is formed into a circumferential shape, so there is the advantageous effect of reduction of the possibility of fracture due to the chewing force or other load.

In the dental implant (artificial dental root) which is shown in FIG. 4A and FIG. 4B, reference notation 1 indicates the main body (core member). This is formed into a circular column shape from a titanium material, titanium alloy material, etc. Further, the screw part 2 is comprised of a first screw part 21 and a second screw part 22. In the first screw part 21, the groove 24 of the thread has a shape which becomes gradually shallower in the direction of the dental prostheses abutment (support part) 6 and becomes equal to the surface of the main body 1. Further, in the second screw part 22, the depth of the groove of the thread is constant. Note that, the front end of the screw part 2 which strikes the bottom part of the main body 1 being formed into a spherical surface shape is sometimes preferable from the viewpoint of easing the effect on the body at the time of placement.

The screw part 2 need only be formed with a groove 24 which becomes shallower in the direction of the support part. It is not necessarily required to form the screw part divided into first and second parts as illustrated.

The screw part 2 has cut surfaces 8. The cut surfaces 8 have the action of preventing rotation of the main body 1. The cut surfaces 8 are preferably provided at the facing surfaces.

Further, setting the outside diameter 23 of the screw part 2 to equal the diameter 11 of the main body or less than that is sometimes preferable from the viewpoint of streamlining the surgical placement. A lineup of parts is made available to enable the length of the screw part 2 (first screw part 21+second screw part 22) and the length other than the screw part 2 to be suitably selected according to the placement location, but at the very least, the range of placement at the alveolar bone is preferably clarified in accordance with the arrangement of the recrystallized ceramic covering layer 4 and the hydrothermal synthesized coating 5.

The dental prostheses abutment 3 is, in the case of a one-piece type, the part for attachment of the crown and, in the case of a two-piece type, the part forming the upper structure. For example, it is provided with a shape which converges in the upper direction of the main body. The front end of the dental prostheses abutment 3 has a flat shape as shown in FIG. 4A and FIG. 4B, but that shape may be suitably changed depending on the shape of the crown etc.

The recrystallized ceramic covering layer 4 is formed by the above-mentioned technique from recrystallized hydroxyapatite or other calcium phosphate. The layer is in a state of a roughened surface and a state with greater thickness than the diameter of the main body.

The hydrothermal synthesized coating 5 is a part which is obtained by buffing or otherwise treating the surface by a polishing material to render it a mirror surface. It mainly contacts the gum. The hydrothermal synthesized coating 5 is furthermore formed as a hydrothermal synthesized coating 5 which is provided with a predetermined thickness by hydrothermal synthesis treatment using an aqueous solution which contains α-TCP, β-TCP, or other calcium phosphate compound, a water vapor atmosphere, etc. and in a state of supersaturated concentrations of the phosphate salt and calcium salt. Note that, the hydrothermal synthesized coating is a thin one of several nanometers or so, so the brightness of the mirror surface is not impaired and the coating becomes a reference for the depth of placement of the implant when placing it in the alveolar bone. The hydrothermal synthesized coating is formed by the above-mentioned hydrothermal synthesis treatment, so a phosphorus component and calcium component are contained. Furthermore, the hydrothermal synthesized coating is formed in the form of synthesized hydroxyapatite and its crystals whereby bonding with the gum is promoted.

Further, the dental prostheses abutment 6 is comprised of a part for fastening the dental prostheses, that is, the crown, and a gum contact part. The alveolar bone implant part 7 has the same range as the recrystallized ceramic covering layer 4.

FIG. 4A shows the state where the core member has been processed but the covering layer has still not been formed. The dental implant differs in shape between the front teeth and the back teeth. Further, depending on the state of the oral cavity of a patient, the length of the screw part 2 may be left as it is or may be lengthened or shortened or may be made thicker or thinner. A large number of ones with different dimensions are preferably prepared.

The example which is shown in FIG. 4A and FIG. 4B and FIG. 5 shows a one-piece type of dental implant which is close to a cylindrical shape overall. The present invention includes the form of a two-piece type of dental implant as well, but a type like the one-piece type which connects the inside and outside of the body in a state with the top part sticking out from the gum right after placement is suitable.

The example which is shown in FIG. 4A and FIG. 4B and FIG. 5, compared with the example which is shown in FIG. 3, is a column shaped member with little relief shapes such as shown in FIG. 4A. Furthermore, there are sometimes a large number of implants with different dimensions, so again it is unclear to exactly what depth to place the implant in the alveolar bone. Further, there are actually also implants which are treated on their surfaces or covered by other techniques. The obtained recrystallized ceramic covering layer may also differ in color from the surroundings and differ in state, but basically due to the inherent color of the metal, in most cases it does not stand out or does not serve as an indicator of from where to where the alveolar bone implant part is formed. However, in the present invention, the shapes and colors of the recrystallized ceramic covering layer and hydrothermal synthesized coating enable the position of placement in the jawbone to be clearly shown.

FIG. 4B shows the recrystallized ceramic covering layer 4 and hydrothermal synthesized coating 5 actually applied to the core member of FIG. 4A. As shown in FIG. 4B as well, the columnar main body 1 is formed with a recrystallized ceramic covering layer 4, but by crystal growth by recrystallization, overall a thickness is provided. The alveolar bone implant part 7 can be recognized at one glance.

The hydrothermal synthesized coating 5 is formed with the synthesized coating, but it is smooth in state and shines silver in state. It is sufficient to form it at the contact surface with the gum. Even if formed above that, the width of the hydrothermal synthesized coating 5 is not particularly limited so long as attachment of the crown (dental prostheses) etc. is not obstructed.

### [Example of Surgical Placement of Implant]

FIG. 6 is a schematic view which shows the state of fastening the dental implant which is shown in FIG. 4B and FIG. 5 to the alveolar bone and attaching the dental prostheses.

In the case of the illustrated example, a hole is drilled in the alveolar bone 31 by a tool, then the screw part 2 of the dental implant shown in FIG. 4B which is provided with the recrystallized ceramic covering layer 4 is screwed into the hole for attaching it. At the time of this fastening work, if, in the state with a hole drilled in the alveolar bone 31, inserting the screw part 4 of the recrystallized ceramic covering layer 4 to an extent of screwing it in, the residue will be ejected in the direction of the dental prostheses abutment (support part) 6.

The implant is in a state implanted up to the boundary of the recrystallized ceramic covering layer 4 and the hydrothermal synthesized coating 5. It may in some cases be preferable to drill the hole in the alveolar bone based on the width of the recrystallized ceramic covering layer 4, that is, the length of the alveolar bone implant part 7.

The gum part 32 need not match the range of the hydrothermal synthesized coating 5, but it preferably is made at least wider than the gum. Further, the crown (dental prostheses) 33 changes in shape according to whether it is a front tooth or a back tooth and is suitably selected in accordance with the treatment location.

### Example 1

In the present example, the production of the dental implant 1 which was previously explained with reference to FIG. 3 will be explained.

As the starting material, a composite material which contained melting point 1720°C α-tricalcium phosphate powder and melting point 1630°C hydroxyapatite powder, respectively synthesized by wet synthesis, mixed to give hydroxyapatite powder in 5% to 9% of the total weight was obtained.

On the other hand, for use as a core member, pure titanium (titanium metal) was formed into a predetermined shape, then the portion for placement in the alveolar bone was sandblasted to roughen the surface of the core member. Further, the contact surface with the gum was polished to remove fine relief shapes from that part.

After this, the sandblasted surface was sprayed with plasma based on the melting point of the hydroxyapatite in accordance with an ordinary method to form a spray-deposited covering layer. The thickness of the obtained spray-deposited covering layer was about 30 µm.

After forming the spray-deposited covering layer, hydrothermal treatment was performed under different conditions within the scope of the present invention. For performing the hydrothermal treatment, the core member was dipped in a pH 5.5 to 12 calcium phosphate aqueous solution for 9 hours to 28 hours in a state of a temperature of 110°C to 125°C and pressure of 0.1 to 0.2 MPa. The surface condition of the obtained recrystallized ceramic covering layer was observed using an electron microscope, whereupon as explained previously with reference to FIG. 1, FIG. 2A, and FIG. 2B, an implant surface of a mixed state of needle shaped and/or hexagonal column shaped hydroxyapatite crystals grown to lengths of 2 to 7 µm or so aligned in one direction and state of these extending radially with respect to the implant surface was obtained. From this, it was learned that in the case of the present example, the targeted dental implant was obtained.

### Example 2

The technique described in the above Example 1 was followed to produce an implant similar to the dental implant 1. However, in the present example, the following manufacturing conditions were applied to produce the targeted implant.

As the starting material, a composite powder obtained by drying and granulating a slurry prepared so as to give melting point 1620°C hydroxyapatite in 1% to 15% with respect to the total weight when synthesizing melting point 1700°C β-tricalcium phosphate by wet synthesis was obtained.

On the other hand, for use as a core member, pure titanium (titanium metal) was formed into a predetermined shape, then the portion for placement in the alveolar bone was sandblasted to roughen the surface of the core member. Next, plasma spraying based on the melting point of hydroxyapatite was performed by an ordinary method to form the spray-deposited covering layer. The thickness of the obtained spray-deposited covering layer was about 45 µm. In the case of the present implant, the contact surface of the core member with the gum was polished to a mirror surface in advance by roller burnishing.

After forming the spray-deposited covering layer, hydrothermal treatment was performed under different conditions within the scope of the present invention. For performing the hydrothermal treatment, the core member was dipped in a pH 5.5 to 12 calcium phosphate aqueous solution for 9 hours to 28 hours in a state of a temperature of 110°C to 125°C and pressure of 0.1 to 0.2 MPa. The surface condition of the obtained recrystallized ceramic covering layer was observed using an electron microscope, whereupon as explained previously with reference to FIG. 1, FIG. 2A, and FIG. 2B, a dental implant having an implant surface of a mixed state of needle shaped and/or hexagonal column shaped hydroxyapatite crystals grown to lengths of 2 to 7 µm or so aligned in one direction and state of these extending radially with respect to the implant surface was obtained. Further, it was learned that an implant surface was formed which was finally covered by regions of hydroxyapatite crystals aligned in the same direction and regions where the crystals are arranged radially from the implant surface in the outer direction as shown in these figures. From this, it was learned that in the case of the present example, the targeted dental implant was obtained.

### Example 3

The technique described in the above Example 1 was followed to produce an implant similar to the dental implant 1. However, in the present example, the following manufacturing conditions were applied to produce the targeted implant.

As the starting material, a composite powder was obtained by respectively synthesizing melting point 1680°C α-tricalcium phosphate powder and melting point 1600°C hydroxyapatite powder by wet synthesis, then mixing them to give hydroxyapatite powder in 5% to 15% of the total weight.

On the other hand, for use as a core member, pure titanium (titanium metal) was formed into a predetermined shape, then the portion for placement in the alveolar bone was sandblasted to roughen the surface of the core member. Next, plasma spraying was performed based on the melting point of hydroxyapatite by an ordinary method to form the spray-deposited covering layer. The thickness of the obtained spray-deposited covering layer was about 40 µm. In the case of the present implant, the contact surface of the core member with the gum was polished to a mirror surface in advance (buffed with cloth).

After forming the spray-deposited covering layer, hydrothermal treatment was performed under different conditions within the scope of the present invention. For performing the hydrothermal treatment, the core member was dipped in 4 cc to 8 cc of water per dental implant for 9 hours to 28 hours in a state of a temperature of 110°C to 125°C and pressure of 0.1 to 0.2 MPa. As a result, in the case of the present example, by reducing the amount of water, at the time of hydrothermal treatment, the molten hydroxyapatite dissolved out into the water and formed a locally supersaturated aqueous solution which contained a phosphate component and calcium component.

The surface conditions of the obtained recrystallized ceramic covering layer were observed using an electron microscope, whereupon as explained previously with reference to FIG. 1, FIG. 2A, and FIG. 2B, it was learned that a dental implant which has an implant surface of a mixed state of needle shaped and/or hexagonal column shaped hydroxyapatite crystals grown to lengths of 2 to 7 µm or so aligned in one direction and state of these extending radially with respect to the implant surface was obtained. From this, it was learned that in the case of the present example, the targeted dental implant was obtained.

### [Evaluation Test]

To evaluate the placement strength of the dental implant (artificial dental root) which was produced in the above Example 3, the extraction strength (MPa) after placing the artificial dental root in the bone tissue was measured. Note that, the samples of implants which were used in this example were all produced under the same manufacturing conditions.

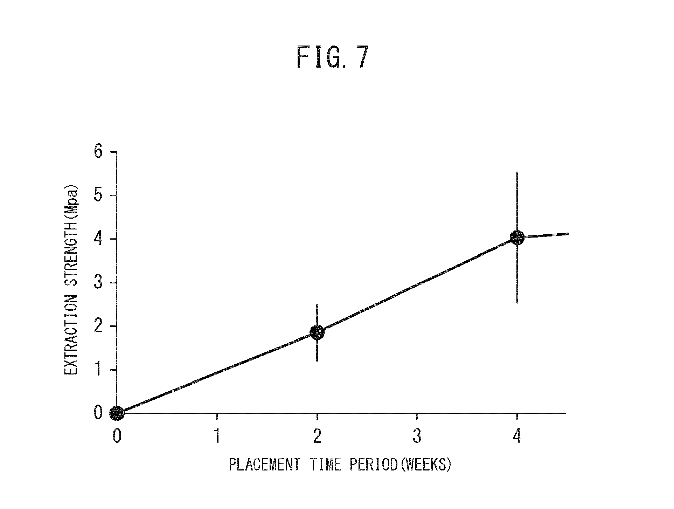

Holes for implants were drilled at predetermined positions of the femurs and alveolar bones of adult dogs (two locations each) by a commercially available drilling tool. Next, samples of implants were placed in the holes and allowed to stand for four weeks. During the four week standing period, the change in the extraction strength along with the elapse of time was investigated by using an Instron type tensile tester to measure the extraction strength two weeks after placement and four weeks after placement, that is, two times. The measurement was performed by measuring the strength when extracting the implants from the embedded positions of the jawbone and femur. The measured extraction strength (MPa), when taking the average value, as plotted in FIG. 7, was about 2 MPa after two weeks and about 4 MPa after four weeks.

From the results of measurement which are shown in FIG. 7, it could be confirmed that in the fourth week, the extraction strength is improved to an extent enabling a crown to be attached and bonding with the bone tissue is promoted. Further, the improvement of the strength such as shown in FIG. 7 is deemed to be due to the increase in the degree of chemical bonding due to the hydrogen bonds, that is, the increase in the biological bond strength.

### Industrial Applicability

According to the present invention, a dental implant which has an implant surface comprised of aligned crystal groups of length about 2 to 7 µm needle shaped and/or column shaped hydroxyapatite crystals aligned in the same direction and/or radial crystal groups of crystals extending radially from the implant surface to the outside direction is provided. By using such an implant for surgical placement, a bone-forming action arises from the implant surface. This quickly stabilizes after surgery. For example, in about 4 weeks after surgical placement, a bonding strength of an extent enabling attachment of the crown can be obtained, so there is great possibility of utilization as a dental implant in the industry. Further, together with this, at the surface of the implant main body which contacts the gum, a coating which is able to be formed by crystals of calcium phosphate is formed as a hydrothermal synthesized coating, so the bondability with the gum becomes good. Furthermore, a dental implant which is stable over a long period of time can be provided. The value of use in the field of dental implants is extremely high.

### Reference Notations List

1. main body
2. screw part
3. dental prostheses abutment
4 recrystallized ceramic covering layer
5. hydrothermal synthesized coating
6. dental prostheses abutment (support part)
7. alveolar bone implant part
8. cut surface

## Claims

1. A dental implant which comprises a columnar core member and a covering layer which is covered over at least part of the outer circumferential surface of the core member and which is comprised of a recrystallized apatite-based ceramic, wherein said covering layer is a hydrothermal treated layer of a plasma spray-deposited covering which is formed by plasma spraying a composite which contains two or more types of calcium phosphate-based compounds which differ in melting point on an outer circumferential surface of said core member based on a melting point of a low melting point calcium phosphate-based compound in said calcium phosphate-based compounds.

2. The dental implant according to claim 1 which further comprises a hydrothermal synthesized coating which is derived from a supersaturated state aqueous solution in which phosphate ions and calcium ions are coexistent or calcium phosphate aqueous solution at the outer circumferential surface of said core member.

3. The dental implant according to claim 1 or 2, wherein said covering layer is comprised of length 2 µm to 7 µm needle shaped and/or column shaped crystals of calcium phosphate-based compounds in aligned crystal groups which are comprised of said crystals formed aligned in a certain direction and/or radial crystal groups where they are formed extending from the surface toward the outer direction covering the surface.

4. The dental implant according to any one of claims 1 to 3, wherein said implant is comprised of parts which are formed by working said core member including an alveolar bone implant part which is provided with a screw part which has cut surfaces and a dental prostheses abutment and wherein said alveolar bone implant part has said covering layer at least at the region other than said screw part.

5. The dental implant according to claim 4, further comprising, between said alveolar bone implant part and said dental prostheses abutment, a columnar gum contact part which is formed in a range of said core member which contacts the gum and which does not have relief shapes, said gum contact part having a polished surface on its outer circumferential surface and a hydrothermal synthesized coating which is formed on this polished surface and which has a composition which contains a phosphorus component, calcium component, and combined components of the same and is the same as or different from said hydrothermal treated layer.

6. The dental implant according to claim 5, wherein said hydrothermal synthesized coating contains hydroxyapatite crystals.

7. The dental implant according to claim 5 or 6, wherein said hydrothermal synthesized coating is a coating which is formed by hydrothermal treatment which dips said core member after formation of said plasma spray-deposited covering in a supersaturated state aqueous solution in which phosphate ions and calcium ions are coexistent or a solution in which calcium phosphate is dipped.

8. The dental implant according to any one of claims 1 to 7, wherein said core member which supports said covering layer has a rough surface structure which is formed in advance on its surface.

9. A method of production of a dental implant including a columnar core member and a covering layer which is covered over at least part of the outer circumferential surface of said core member and which is comprised of a recrystallized apatite-based ceramic, comprising forming said covering layer by
plasma spraying a composite which contains at least two types of calcium phosphate-based compounds with different melting points on the outer circumferential surface of said core member based on the melting point of a low melting point calcium phosphate-based compound in said calcium phosphate-based compounds and
hydrothermally treating said plasma spray-deposited covering which is formed by the plasma spray step to form a hydrothermal treated layer.

10. The method of production according to claim 9 further comprising a step of forming a hydrothermal synthesized coating on the outer circumferential surface of said core member using a supersaturated state aqueous solution in which phosphate ions and calcium ions are coexistent or a calcium phosphate aqueous solution.

11. The method of production according to claim 9 or 10 further comprising
performing said plasma spraying by bringing a powder of said composite into contact with a high temperature plasma flame and spraying molten state composite on said core member and
performing said hydrothermal treatment by dipping or exposing said core member which is provided with the plasma spray-deposited covering in water, an aqueous solution in which phosphate ions and calcium ions are coexistent, a calcium phosphate aqueous solution, or an atmosphere of a vapor of the same.

12. The method of production according to any one of claims 9 to 11 further comprising a core member forming step of forming in advance an alveolar bone implant part which is provided with a screw part which has cut surfaces and a dental prostheses abutment at said core member and forming said covering layer at least at the region of said alveolar bone implant part other than said screw part.

13. The method of production according to claim 12 further comprising a step of forming, between said alveolar bone implant part and said dental prostheses abutment, a columnar gum contact part which does not have relief shapes in a range of said core member which contacts the gum,
said step including forming a polished surface at an outer circumferential surface of said gum contact part and forming on said polished surface a hydrothermal synthesized coating which has a composition which contains a phosphorus component, calcium component, and combined components of the same and is the same as or different from said hydrothermal treated layer.
